# EUROPEAN PATENT SPECIFICATION

(11) **EP 0 564 461 B1**
(45) Date of publication and mention of the grant of the patent: **02.01.1997**
(21) Application number: 91918565.2
(22) Date of filing: 30.10.1991
(51) Int. Cl.: A61K 39/395

(54) **THE USE OF ANTIBODIES TO TNF OR FRAGMENTS DERIVED THEREOF AND XANTHINE DERIVATIVES FOR COMBINATION THERAPY AND COMPOSITIONS THEREFOR**
VERWENDUNG VON ANTIKÖRPERN GEGEN TNF ODER DIE DAVON ABGELEITETEN FRAGMENTE UND XANTHINDERIVATE IN DER KOMBINATIONSTHERAPIE UND ZUSAMMENSETZUNGEN DAZU
UTILISATION D'ANTICORPS CONTRE LE TNF, OU DE FRAGMENTS DERIVES DE CEUX-CI, AINSI QUE DE DERIVES XANTHIQUES POUR LA THERAPIE D'ASSOCIATION, ET COMPOSITIONS ASSOCIEES

(30) Priority: 01.11.1990 GB 9023783
(43) Date of publication of application: 13.10.1993
(73) Proprietor: CELLTECH THERAPEUTICS LIMITED, Slough, Berkshire SL1 4EN (GB); HOECHST AKTIENGESELLSCHAFT, 65926 Frankfurt am Main (DE)
(72) Inventor: ANAGNOSTOPULOS, Hiristo, D-6200 Wiesbaden 1 (DE); GEBERT, Ulrich, D-6246 Glashutten 2 (DE); HANEL, Heinz, D-6370 Oberursel (DE); LIMBERT, Michael, D-6238 Hofheim (DE); BODMER, Mark, William Rose Cottage 5 Manor Road, Oxford OX1 5AS (GB); HIGGS, Gerald, Anthony, London SE21 7JU (GB)
(74) Representative: Hallybone, Huw George
(86) International application number: GB9101907
(87) International publication number: WO9207585

(56) References cited:
- Clinics in Chest Medicine, vol. 11, no. 4, December 1990, W.B. Saunders Co., Harcourt Brace Jovanovich, Inc.; G. Goldstein et al.: "Pharmacologic treatment of the adult respiratory distress syndrome", pages 773-787, see pages 783,784: summary
- American Review of Respiratory Disease, vol. 137, no. 4, part 2, suppl. April 1988, Am. Lung Ass. New York (US), C.M. Lilly et al.: "Pentoxyfylline prevents hypotension and pulmonary injury caused by tumor necrosis factor", page 138, see the whole abstract
- Biochemical and Biophysical Research Communications, vol. 155, no. 3, 30 September 1988, New York (US), R.M. Strieter et al.: "Cellular and molecular regulation of tumor necrosis factor-alpha production by pentoxifylline", pages 1230-1236, see the whole article
- Nature, vol. 330, 17 December 1987, London (GB), K.J. Tracey et al.: "Anti- cachectin/TNF monoclonal antibodies prevent septic shock during lethal bacteraemia", pages 662-664, see the whole abstract

## Description

### Field of the Invention

The present invention relates to a pharmaceutical product for the treatment of conditions associated with elevated levels of tumour necrosis factor-α (herein referred to as TNF) and to the manufacture of such a product. The pharmaceutical product may, for example, be employed in the treatment of sepsis, and, in particular in the treatment of septic, or endotoxic shock.

### Background to the Invention

TNF is a cytokine which is produced by activated macrophage and other cells and is an important regulator in inflammation and immunity. It is implicated in septic shock - an often fatal condition associated with Gram-negative or Gram-positive bacteremia - as well as in other conditions such as adult respiratory distress syndrome, graft-versus-host disease and auto-immune diseases.

Treatments of these conditions involving the use of antibodies to TNF (anti-TNF antibodies) have been proposed and tested. For example, Beutler et al (Science (1985), 229, 869-871) showed that passive immunisation with a rabbit polyclonal antiserum against TNF protected mice from the lethal effects of Gram-negative endotoxin. Similarly, antibody therapy against TNF has been shown to decrease mortality in mice undergoing graft-versus-host disease (GVHD) and to prevent splenomegaly, and cutaneous and intestinal lesions associated with acute phase GVHD. (Piguet, P.F., et al, J. Exp. Med. 1987; 166: 1280; Shalaby, M.R., et al, Transplantation 1989; 47: 1057).

In addition, patent applications of Celltech Limited disclose the use of anti-TNF antibodies in the amelioration of side effects associated with anti-lymphocyte therapy of graft-rejection (WO89/08460), and with anti-neoplastic chemotherapy (WO89/01950).

Certain xanthine derivatives are also known to be inhibitors of TNF. For example, EP-A-0344586 of Hoechst Aktiengesellschaft discloses xanthine derivatives effective in inhibiting the TNF whose release is induced by certain TNF-releasing substances such as amphoterocin B.

The present inventors have observed that in some experimental models of septic shock a surprising combination effect is produced if an antibody to TNF and a xanthine derivative are used in combination.

### Summary of Aspects of the Invention

Thus, according to a first aspect of the present invention there is provided a pharmaceutical product comprising an antibody to TNF or a TNF binding fragment thereof and a xanthine derivative as a combined preparation for simultaneous combined, simultaneous separate, or sequential use in therapy.

Such a pharmaceutical product may take the form of a pharmaceutical composition in which the antibody to TNF, or TNF binding fragment thereof and the xanthine derivative occur in admixture, optionally together with a pharmaceutically acceptable excipient, diluent, or carrier.

The ratio by weight of xanthine derivative to anti-TNF antibody in the composition may vary between 450: 1 and 1:10; preferably it is in the range 150:1 - 1:5 and particularly preferably between 30:1 and 1:2 for example 1:1.

Suitable xanthine derivatives for inclusion in the pharmaceutical product of the present invention include the following:
1) Compounds of the formula I 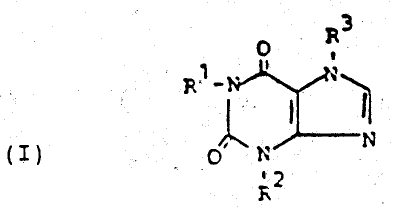 in which one of the radicals R¹ and R³ represents a straight-chain alkyl, (ω-1)-oxoalkyl or (ω-1)-hydroxyalkyl group having 3 to 8 carbon atoms, and the two other radicals, R² and R³ or R¹ and R², represent straight-chain or branched alkyl groups having 1 to 8 carbon atoms in the position of R¹ and R³ and 1 to 4 carbon atoms in the position of R², where the total of carbon atoms in these two alkyl substituents does not exceed 10;
2) Compounds of the formula II 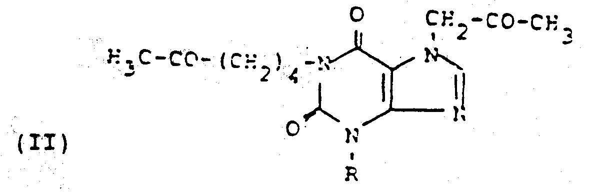 in which R represents an alkyl radical having 1 to 4 carbon atoms;
3) Compounds of the formula III 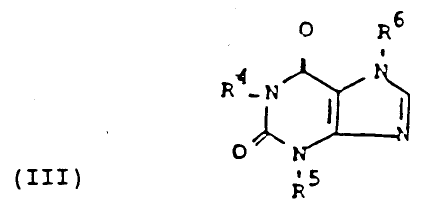 in which at least one of the radicals R⁴ and R⁶ represents a tertiary hydroxyalkyl group of the formula 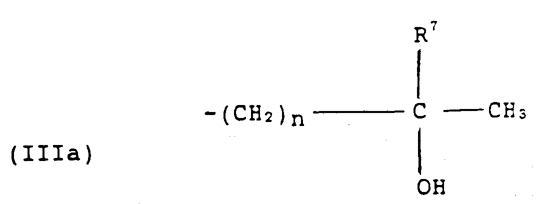 where R⁷ denotes an alkyl group having up to 3 carbon atoms, and n denotes an integer from 2 to 5, and - if only one of the radicals R⁴ or R⁶ denotes such a tertiary hydroxyalkyl group of the formula IIIa - the other radical represents a hydrogen atom or an aliphatic hydrocarbon radical R⁸ which has up to 6 carbon atoms and whose carbon chain can be interrupted by up to 2 oxygen atoms or substituted by an oxo group or up to two hydroxyl groups (in which case an oxo or hydroxyl group present in the radical R⁸ is preferably separated from the nitrogen by at least 2 carbon atoms), and R⁵ represents an alkyl group having 1 to 4 carbon atoms;
4) Prodrug forms of the compounds of the formulae I to III, and/or
5) Metabolites of the compounds of the formulae I to III.

In particularly preferred embodiments the xanthine derivative is of formula I with a hexyl, 5-oxohexyl or 5-hydroxyhexyl group in the position of R¹ or R³. For example, 1-hexyl-3,7-dimethylxanthine, 1-(5-hydroxyhexyl)-3,7-dimethylxanthine, 3,7-dimethyl-1-(5-oxohexyl)xanthine, 7-(5-hydroxyhexyl)-1,3-dimethylxanthine, 1,3-dimethyl-7-(5-oxohexyl)xanthine, 1,3-di-n-butyl-7-(2-oxopropyl or 3-oxobutyl)xanthine, 1-(5-hydroxyhexyl)-3-methyl-7-propylxanthine and 3-methyl-1-(5-oxohexyl)-7-propylxanthine (= propentofylline), and especially 3,7-dimethyl-1-(5-oxohexyl)xanthine (= pentoxifylline) may be included in the pharmaceutical product.

Particularly preferred compounds of the formula III for inclusion in this product are those compounds in which R⁵ represents a methyl or ethyl group. Similarly preferred are those compounds of the formula III in which only one of the two radicals R⁴ or R⁶ represents the tertiary hydroxyalkyl group defined above. Additionally preferred are those compounds in which R⁷ represents a methyl group, and n denotes an integer from 3 to 5, so that the tertiary hydroxyalkyl radical IIIa represents either [(ω-1)-hydroxy-(ω-1)-methyl]-pentyl, -hexyl or heptyl, especially those in which R⁵ denotes methyl or ethyl.

Also worth particular mention are those compounds of formula III in which R⁴ represents the tertiary hydroxyalkyl group, and R⁶ represents hydrogen, alkyl, hydroxyalkyl or alkoxyalkyl, having 1 to 4 carbon atoms in each case, for example, 7-ethoxymethyl -1-(5-hydroxy-5-methylhexyl)-3-methylxanthine, 7-propyl-1-(5-hydroxy-5-methylhexyl)-3-methylxanthine, or 1-(5-hydroxy-5-methylhexyl)-3-methylxanthine.

As mentioned above some embodiments of the pharmaceutical product of the present invention may comprise the oxoalkylxanthines of the formula I and II, or the hydroxyalkylxanthines of the formula I and III, not as such but in the form of a prodrug from which the therapeutically active xanthine compounds, having the substituents defined in formulae I, II and III, can be released only by biotransformation in the body. Suitable derivatives include the acetalized oxoalkylxathines in which the carbonyl groups are replaced by the structural element of formula IV 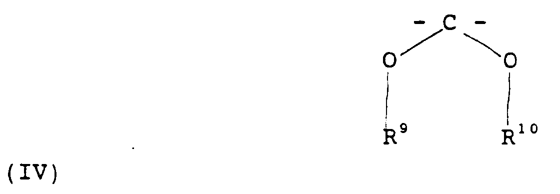 and the O-acylated hydroxyalkylxanthines having the structural element of the formula (V)

R¹¹ - CO - O (V)

in place of the hydroxyl group, where R⁹ and R¹⁰ each represents an alkyl group having up to 4 carbon atoms or together represents an ethylene, trimethylene or tetramethylene group, and R¹¹ denotes an alkyl radical having up to 4 carbon atoms or optionally substituted phenyl or pyridyl.

In particular the preferred xanthine derivatives for inclusion in the pharmaceutical product of the present invention are pentoxifylline (3,7-dimethyl-1-(5-oxohexyl)xanthine), also known as Trental, and 1-(5-hydroxy-5-methylhexyl)-3-methyl xanthine which is referred to hereafter as HWA 138.

The anti-TNF antibody or TNF binding fragment thereof included in the pharmaceutical product of the present invention is preferably a TNF neutralising antibody or antibody fragment. By neutralisation is intended the reduction in, or inhibition of a biological activity of TNF as measured by an in vitro or in vivo test.

The anti-TNF antibody or fragment included in the pharmaceutical product of the present invention may in general belong to any immunoglobulin class. Thus for example the anti-TNF antibody may be an immunoglobulin G or immunoglobulin M antibody.

The anti-TNF antibody may be of animal, for example mammalian origin and may be for example of murine, rat or human origin. The antibody may be a whole immunoglobulin, or a fragment thereof, for example a fragment derived by proteolytic cleavage of a whole antibody, such as F(ab')₂, Fab' or Fab fragments, or fragments obtained by recombinant DNA techniques, for example Fv fragments (as described in International Patent Application No. WO89/02465).

The anti-TNF antibody may be polyspecific but is preferably monospecific for human TNF. The antibodies may be polyclonal or monoclonal antibodies. Particularly useful antibodies for use according to the invention include recombinant anti-TNF antibodies and fragments thereof, i.e. anti-TNF antibodies or fragments which have been produced using recombinant DNA techniques.

Especially useful recombinant antibodies include, (1) those having an antigen binding site at least part of which is derived from a different antibody, for example those in which hypervariable or complementarity determining regions of one antibody have been grafted into variable framework regions of a second, different, and preferably human, antibody (as described in European Patent Application EP-A-239400); (2) recombinant antibodies or fragments wherein non-Fv sequences have been substituted by non-Fv sequences from other, different antibodies (as described in European Patent Applications EP-A-171496, EP-A-173494 and EP-A-194276); or (3) recombinant antibodies or fragments possessing substantially the structure of a natural immunoglobulin but wherein the hinge region has a different number of cysteine residues from that found in the natural immunglobulin, or wherein one or more cysteine residues in a surface pocket of the recombinant antibody of fragment is in the place of another amino acid residue present in the natural immunoglobulin (as described in International Patent Applications Nos. WO89/01974 and WO89/01782 respectively).

The anti-TNF antibodies may be prepared using well-known immunological techniques employing TNF as antigen. Thus, for example, any suitable host may be injected with TNF and the serum collected to yield the desired polyclonal anti-TNF antibody after appropriate purification and/or concentration, (for example by affinity chromatography using immobilized TNF as the affinity medium). Alternatively, splenocytes or lymphocytes may be recovered from the TNF-injected host and immortalized using for example the method of Kohler et al, Eur. J. Immunol. 6, 511, (1976), the resulting cells being diluted and cloned to obtain a monoclonal line producing anti-TNF antibodies in accordance with conventional practice. Antibody fragments may be produced using conventional techniques, for example by enzymatic digestion of whole antibodies e.g. with pepsin [Parham, J. Immunol., 131, 2895, (1983)] or papain [Lamoyi and Nisonoff, J. Immunol. Meth., 56, 235, (1983)].

Where it is desired to produce recombinant anti-TNFα antibodies these may be produced using, for example, the general methods described in the above-mentioned patent specifications.

According to a second aspect of the invention there is provided the use of an antibody to TNF or a fragment thereof and of a xanthine derivative in the manufacture of a pharmaceutical product of the first aspect of the invention.

For example, an antibody to TNF and a xanthine derivative as described above may be mixed together and a pharmaceutically acceptable excipient, diluent, or carrier may optionally also be mixed in.

The pharmaceutical product may be utilized in any therapy where it is desired to reduce the level of TNF present in the human or animal body. The TNF may be in circulation in the body or present in an undesirably high level localized at a particular site in the body.

For example, elevated levels of TNF are implicated in immunoregulatory and inflammatory disorders and in septic, or endotoxic, and cardiovascular shock. The pharmaceutical product according to the first aspect of the present invention may be utilized in therapy of conditions which include sepsis, septic or endotoxic shock, cachexia, adult respiratory distress syndrome, AIDS, allergies, psoriasis, T.B., inflammatory bone disorders, blood coagulation disorders, burns, rejection episodes following organ or tissue transplant and autoimmune disease e.g. organ specific disease such as thyroiditis or non-specific organ diseases such as rheumatoid and osteo-arthritis.

Additionally, the pharmaceutical product may be used to ameliorate side effects associated with TNF generation during neoplastic therapy and also to eliminate or ameliorate shock related symptoms associated with the treatment or prevention of graft rejection by use of an antilymphocyte antibody, or may be used for treating multi-organ failure (MOF).

The pharmaceutical product according to the first aspect of the invention is preferably for treatment of sepsis, or septic/endotoxic shock.

The pharmaceutical product according to the first aspect of the invention may be for administration in any appropriate form and amount according to the therapy in which it is employed. It may be for prophylactic use, for example where circumstances are such that an elevation in the level of TNF might be expected or alternatively, the product may be for use in reducing the level of TNF after it has reached an undesirably high level or as the level is rising.

The pharmaceutical product may take any suitable form for administration, and, in particular, will be in a form suitable for parenteral administration e.g. by injection or infusion, for example by bolus injection or continuous infusion. Where the product is for injection or infusion it may take the form of a suspension, solution or emulsion of all or each of the components is an oily or aqueous vehicle and it may contain formulatory agents such as suspending, stabilizing and/or dispersing agents.

Alternatively, the product may be in dry form, for reconstitution before use with an appropriate sterile liquid.

Where the anti-TNF and xanthine derivative components of the pharmaceutical product are for separate administration each may be formulated according to conventional practice and the formulation of each component may contain one or more other active ingredients.

In particular, while the anti-TNF antibody is likely to be unsuitable for oral administration such a limitation may not apply to the xanthine derivative.

When the xanthine derivative is for oral administration the formulation may contain, in addition, to the active ingredient, additives such as; starch - e.g. potato, maize or wheat starch or cellulose - or starch derivatives such as microcrystalline cellulose; silica; various sugars such as lactose; magnesium carbonate and/or calcium phosphate. It is desirable that, if the formulation is for oral administration it will be well tolerated by the patient's digestive system. To this end it may be desirable to include in the formulation mucus formers and resins. It may also be desirable to improve tolerance by formulating the xanthine derivative in a capsule which is insoluble in the gastric juices. It may also be preferable to include the xanthine derivative in a controlled release formulation.

A method of treatment of a human or animal subject suffering from or at risk of a disorder associated with an undesirably high level of TNF comprises administering to the subject an effective amount of the pharmaceutical product according to the first aspect of the invention. In particular the human or animal subject may be suffering from, or at risk from, sepsis, or septic or endotoxic shock.

The dose at which each pharmaceutical product will be administered will depend on the nature of the condition to be treated, the degree to which the TNF to be neutralized is, or is expected to be, raised above a desirable level, and on whether the product is being used prophylactically or to treat an existing condition. The dose will also be selected according to the age and condition of the patient.

Thus, for example, where the product is for treatment or prophylaxis of septic shock suitable doses of antibody to TNF lie in the range 0.001-30mg/kg/day, preferably 0.01-10mg/kg/day and particularly preferably 0.1-2mg/kg/day while suitable doses of xanthine derivative lie in the range 0.5 to 100mg/kg/day, preferably in the range from 0.5 to 50mg/kg/day, and particularly preferably from 1 to 30mg/kg/day. In general, the dose may be continued for as long as is necessary to alleviate the condition associated with the undesirably high level of TNF. In particular, it may be desirable to administer the dose of the xanthine derivative continuously by infusion or to repeat the dose of the xanthine derivative at regular, intervals for example of 2 to 24 hours, preferably 6 to 12 hours even if no repeat dose of anti-TNF antibody is administered.

### Brief Description of the Drawings

Embodiments of the invention are described below by way of example and with reference to the accompanying drawings of which:
- Fig 1: shows a plot of mean deaths per experiment against time in an E.coli LPS induced shock model in which pentoxifylline or the hamster anti-mouse TNF antibody TN3 19.12 were administered to subjects of the experiment at various doses:
- ∇,: pentoxifylline, 300mg/kg; x, pentoxifylline, 10mg/kg;
- △,: pentoxifylline, 1mg/kg; □ control; + ,TN3 19.12, 1mg/kg;
- ◇: TN3 19.12, 30mg/kg;
- Fig 2: shows a similar plot of mean deaths per experiment against time in an E.coli LPS induced shock model in which pentoxifylline and/or TN3 19.12 were administered to subjects at a dose of 1mg/kg:
- △: pentoxifylline, 1mg/kg and TN3 19.12, 1mg/kg;
- ◇: pentoxifylline alone 1mg/kg; □ ; control; + , TN3, 19.12, 1mg/kg;
- Fig 3: shows a similar plot to Fig 2 in which mean deaths per experiment are plotted against time for an E.coli LPS induced shock model in which pentoxifylline and/or TN3 19.12 were administered to subjects at the following doses:
A - control, B-1mg/kg pentoxifylline (single dose) and 1mg/kg-TN3 19.13, C-1mg/kg pentoxifylline (dose repeated at 24, 48 and 72 hours) and 1mg/kg TN3 19.13 (single dose)
- Fig 4: shows a block diagram showing percentage inhibition of mortality in a Salmonella abortus equi LPS induced shock model in which various amounts of TN3 19.12 and/or xanthine derivative HWA 138 were administered to galactosamine sensitised and LPS challenged mice;
- Fig 5: shows a similar block diagram in which inhibition of mortality in a salmonelia abortus equi LPS induced shock model is plotted for an experiment in which various amounts of TN3 19.12 and/or the xanthine derivative pentoxifylline were administered to galactosamine sensitized and UPS challenged mice.

In the examples which follow bacterial LPS (lipopolysaccharide) was used to induce septic shock in mice.

### Description of Embodiments of the Invention

### Example 1

In a first experiment outbred mice were divided into groups and received treatment either with a hamster anti-murine TNF antibody TN3 19.12 or with the xanthine derivative, pentoxifylline.

Each group of animals received 60mg/kg E.coli LPS by intravenous injection into a tail vein. This dose of LPS corresponds to the LD90 in mice.

Those groups of animals treated with TN3 19.12 received between 0.1 and 30mg/kg of the antibody by intravenous injection of 0.1ml of liquid into the tail vein 2 hours before LPS administration.

Those groups to be treated with pentoxifylline received between 1mg/kg and 300mg/kg intravenously five minutes prior to LPS administration.

In control experiments animals received an injection of an equivalent volume of saline.

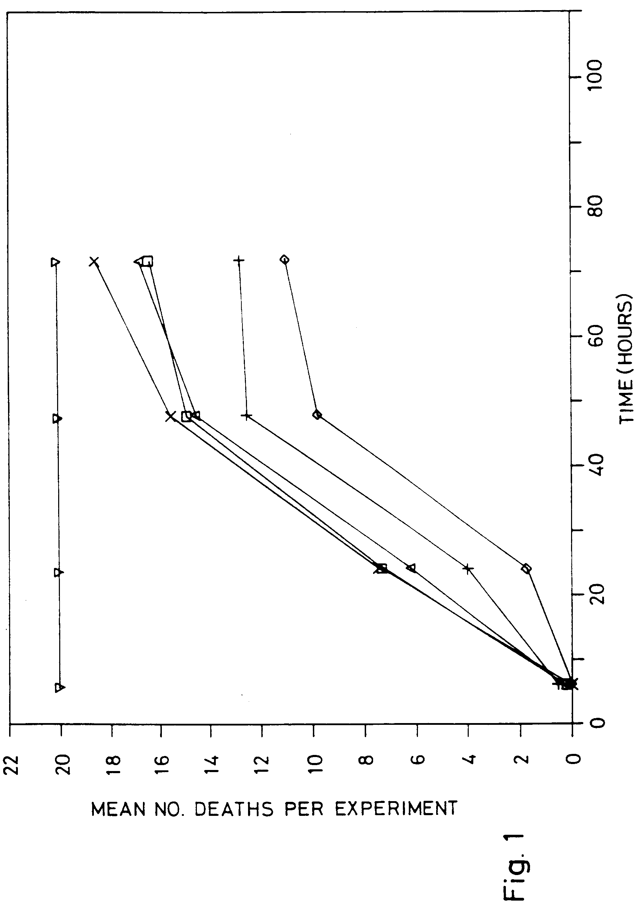

The results of these experiments are shown in Fig 1. TN3 19.12 alone produced a protective effect at doses ≧ 1mg/kg. By contrast, pentoxifylline alone had no effect at doses of 1mg/kg and at higher concentrations it accelerated mortality.

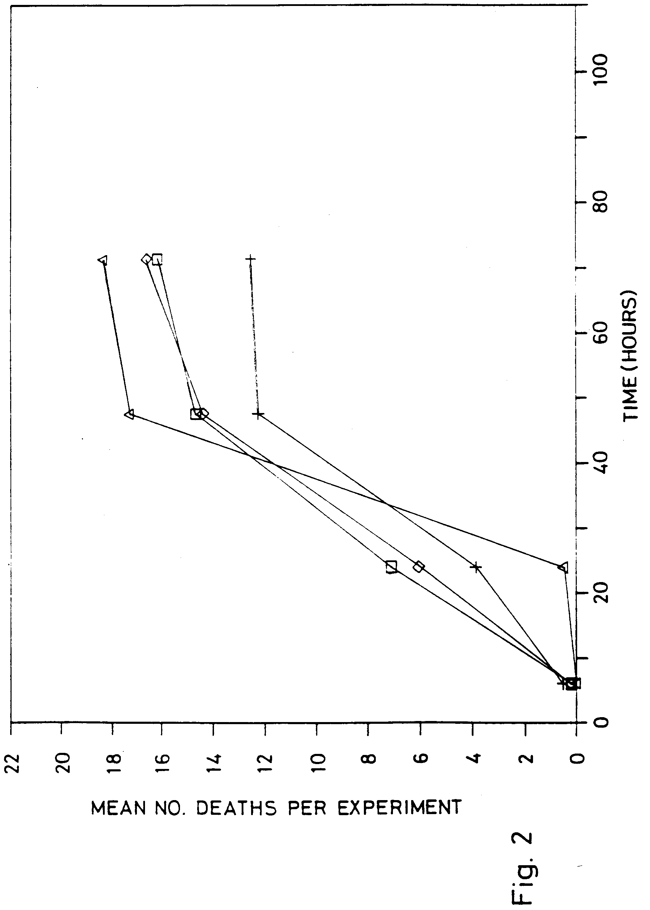

In a second experiment both TN3 19.12 and pentoxifylline were administered at a dose of 1mg/kg, the TN3 antibody being administered 2 hrs prior to LPS and the pentoxifylline 5 mins prior to LPS administration as before. The results are shown in Fig 2 and demonstrate that mice receiving the combined treatment were protected against LPS induced death for 24 hrs after LPS administration. Subsequently, however, mortality rose to levels comparable with the control or pentoxifylline only groups.

It was believed that this effect may be due to the shorter half life of pentoxifylline relative to TN3 and might be compensated for by repeating pentoxifylline administration throughout the treatment period, for example, every 12 or 24 hours.

In order to confirm this hypothesis a further experiment was carried out with groups of twenty mice. The groups received one of three different treatment as follows:
A) Control - received 60mg/kg E.coli LPS intravenously followed by saline injection;
B) Received 1mg/kg TN3 19.12 i.v. 2 hrs prior to challenge with 60mg/kg E.coli LPS i.v. and 1mg/kg pentoxifylline i.v. 5 mins prior to challenge;
C) As B) but these mice also received repeat doses of 1mg/kg pentoxifylline 24,48 and 72 hours after LPS challenge.

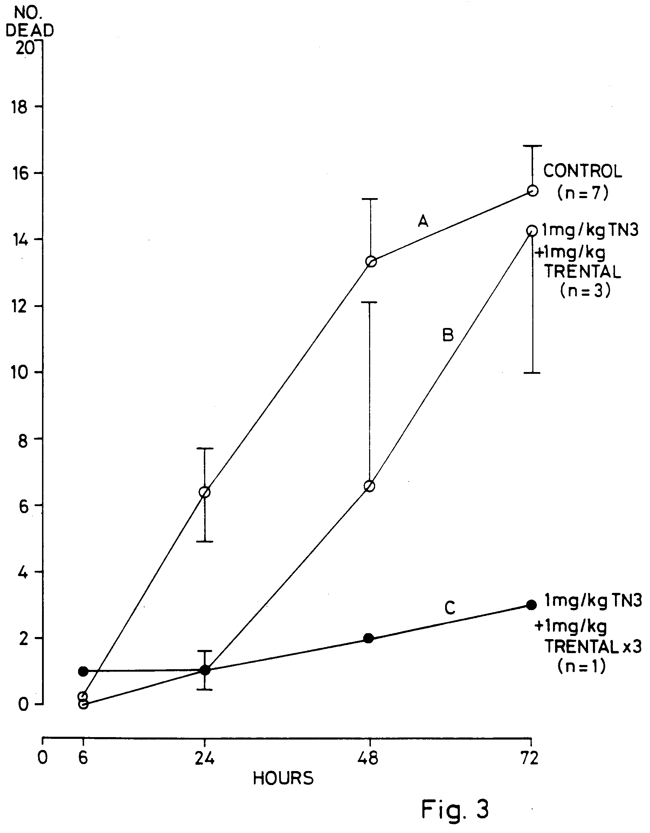

The results of this experiment are shown in Fig 3. As in previous experiments group B) showed enhanced survival relative to the control at 24 hrs from LPS challenge but at later observation times survival was not significantly different from the control. By contrast the survival in group C) was markedly better than the control with 17 out of 20 animals surviving at 72 hrs from LPS challenge.

### Example 2

Similar experiments to those described above were carried out in galactosamine sensitised C57BL mice which were challenged with LPS from Salmonella abortus equi. Mortality was measured 96 hrs after the LPS challenge.

Low doses of TN3 of between 0.1 and 0.25mg/kg administered intravenously 1hr prior to administration of 0.2µg LPS and 10mg galactosamine per mouse had no protective effect. By contrast the xanthine derivative HWA 138 at a dose of 30mg/kg intraperitoneally had a partially protective effect (c.20% inhibition of mortality).

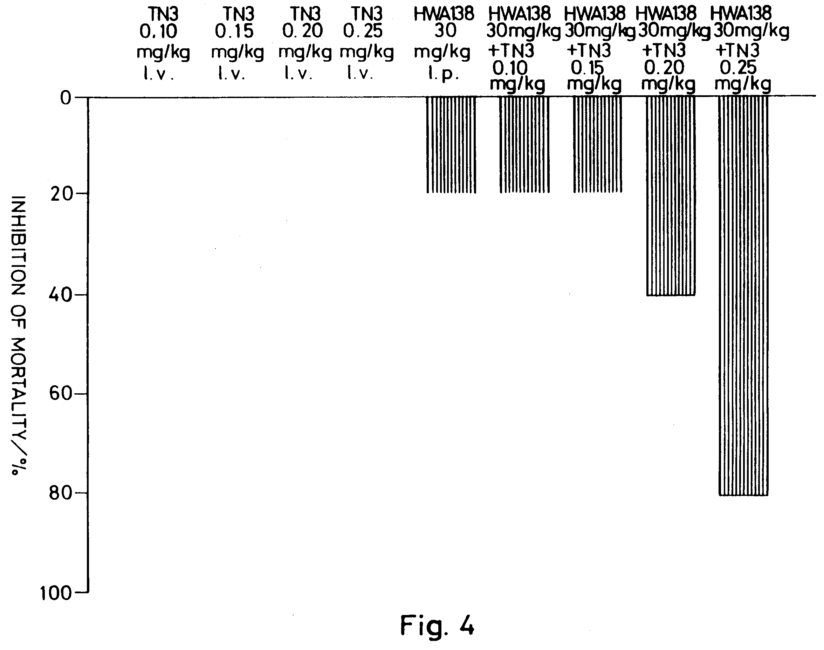

Coadministration of TN3 at doses ≧ 0.2mg/kg together with 30mg/kg HWA 138 significantly reduced mortality relative to 30mg/kg HWA 138 used alone. These results are shown in Fig 4.

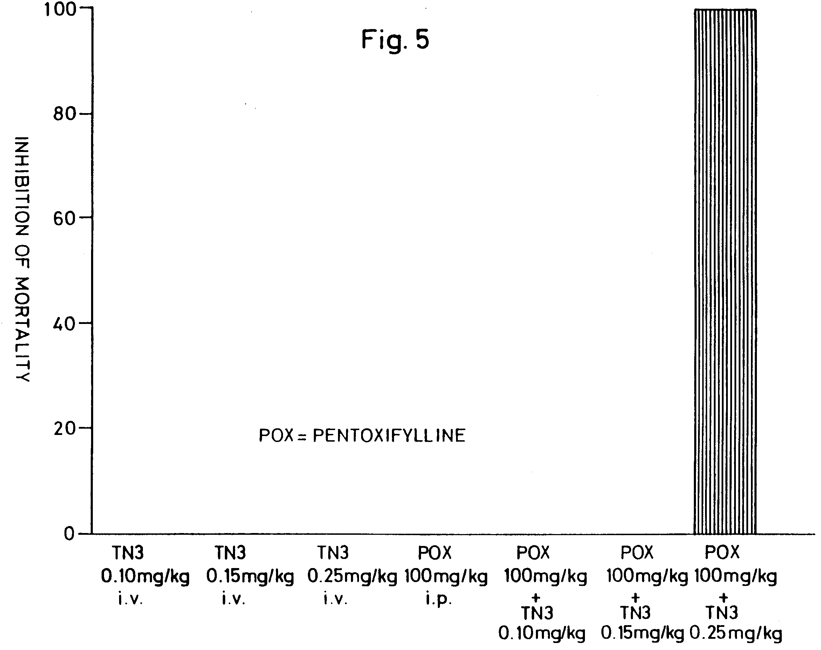

This experiment was repeated using pentoxifylline in place of HWA 138 and similar results were obtained as shown in Fig 5. In this case 100mg/kg of pentoxifylline either alone or in combination with low doses of TN3 (0.10 or 0.15 mg/kg) had no protective effect. However, mortality could be completely inhibited by combined administered of 100mg/kg pentoxifylline and 0.25 mg/kg of TN3.

## Claims

1. A pharmaceutical product comprising an antibody to TNF or a TNF binding fragment thereof and a xanthine derivative as a combined preparation for simultaneous combined, simultaneous separate or sequential use in therapy.

2. A pharmaceutical product according to Claim 1 in the form of a pharmaceutical composition comprising in admixture the antibody to TNF, or TNF binding fragment thereof and the xanthine derivative.

3. A pharmaceutical product according to Claim 2, in which the ratio of xanthine derivative to anti-TNF antibody component is in the range between 450:1 and 1:10 by weight.

4. A pharmaceutical product according to any one of Claims 1-3, in which the xanthine derivative is a compound of formula I. 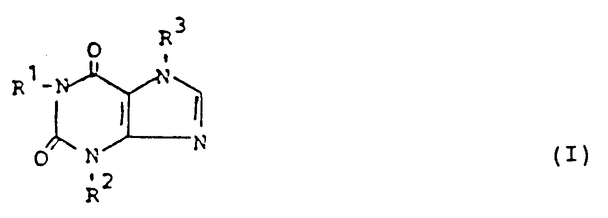 in which one of the radicals R¹ and R³ represents a straight-chain alkyl, (ω-1)-oxoalkyl or (ω-1)-hydroxyalkyl group having 3 to 8 carbon atoms, and the two other radicals, R² and R³ or R¹ and R², represent straight-chain or branched alkyl groups having 1 to 8 carbon atoms in the position of R¹ and R³ and 1 to 4 carbon atoms in the position of R², where the total of carbon atoms in these two alkyl substituents does not exceed 10.

5. A pharmaceutical product according to any one of Claims 1-3, in which the xanthine derivative is a compound of formula II 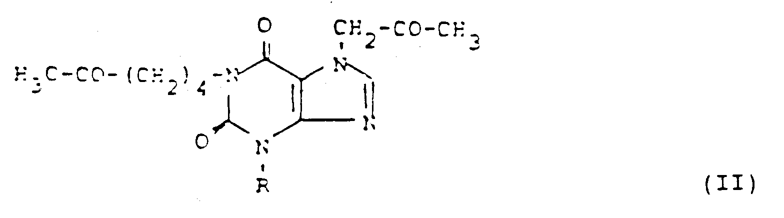 in which R represents an alkyl radical having 1 to 4 carbon atoms.

6. A pharmaceutical product according to any one of Claims 1-3, in which the xanthine derivative is a compound of formula III 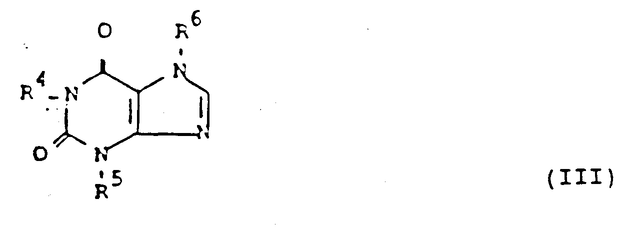 in which at least one of the radicals R⁴ and R⁶ represents a tertiary hydroxyalkyl group of the formula IIIa. 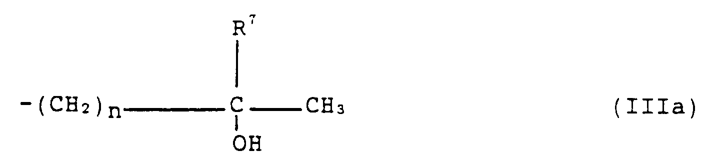 where R⁷ denotes an alkyl group having up to 3 carbon atoms, and n denotes an integer from 2 to 5, and - if only one of the radicals R⁴ or R⁶ denotes such a tertiary hydroxyalkyl group of the formula IIIa - the other radical represents a hydrogen atom or an aliphatic hydrocarbon radical R⁸ which has up to 6 carbon atoms and whose carbon chain can be interrupted by up to 2 oxygen atoms or substituted by an oxo group or up to two hydroxyl groups (in which case an oxo or hydroxyl group present in the radical R⁸ is preferably separated from the nitrogen by at least 2 carbon atoms), and R⁵ represents an alkyl group having 1 to 4 carbon atoms;

7. A pharmaceutical product according to any one of Claims 4, 5 or 6, in which the xanthine derivative is in the form of a prodrug or metabolite of the compound of formula I, II or III.

8. A pharmaceutical product according to Claim 4, in which the xanthine derivative comprises:
1-hexyl-3,7-dimethylxanthine,
1-(5-hydroxyhexyl)-3,7-dimethylxanthine,
3,7-dimethyl-1- (5-oxohexyl)xanthine,
7-(5-hydroxyhexyl)-1,3-dimethylxanthine,
1,3-dimethyl-7-(5-oxohexyl)xanthine,
1,3-di-n-butyl-7-(2-oxopropyl)xanthine,
1,3-di-n-butyl-7-(3-oxybutyl)xanthine,
1-(5-hydroxyhexyl)-3-methyl-7-propylxanthine,
3-methyl-1-(5-oxohexyl)-7-propylxanthine or
3,7-dimethyl-1-(5-oxohexyl)xanthine

9. A pharmaceutical product according to Claim 6, in which the xanthine derivative comprises:
7-ethoxymethyl -1-(5-hydroxy-5-methylhexyl) -3-methylxanthine, 7-propyl-1-(5-hydroxy-5-methylhexyl)-3-methylxanthine, or 1-(5-hydroxy-5-methylhexyl)-3-methylxanthine.

10. A pharmaceutical product according to any one of the preceding claims, in which the anti-TNF antibody or TNF binding fragment thereof is a TNF neutralising antibody or antibody fragment.

11. A pharmaceutical product according to any one of the preceding claims in which the anti-TNF antibody or antibody fragment is monospecific.

12. A pharmaceutical product according to any one of the preceding claims, in which the anti-TNF antibody or antibody fragment is a recombinant antibody or antibody fragment.

13. A pharmaceutical product according to Claim 12, in which the anti-TNF antibody or antibody fragment is a humanized recombinant antibody or antibody fragment.

14. A pharmaceutical product according to any one of the preceding claims for use in the amelioration of side effects associated with TNF generation during neoplastic therapy.

15. A pharmaceutical product according to any one of claims 1-13 for use in the elimination or amelioration of shock related symptoms associated with antilymphocyte therapy.

16. A pharmaceutical product according to any one of claims 1-13 for use in the treatment of multi-organ failure.

17. A pharmaceutical product according to any one of claims 1-13 for use in the treatment of sepsis or septic/endotoxic shock.

18. The use of an antibody to TNF or fragment thereof and of a xanthine derivative in the manufacture of a pharmaceutical product for use as a combined preparation for simultaneous combined, simultaneous separate or sequential use in therapy.

19. A process for the preparation of a pharmaceutical product according to Claim 2 comprising admixing effective amounts of an anti-TNF antibody or fragment thereof and a xanthine derivative.

20. A process according to Claim 19, in which the antibody or fragment thereof and the xanthine derivative are admixed also with a pharmaceutically acceptable excipient, diluent or carrier.

21. The use of the pharmaceutical product of any one of claims 1 to 13 in the manufacture of a composition for the treatment of a disorder associated with an undesirably high level of TNF.

## Patentansprüche

1. Pharmazeutisches Produkt, das einen Antikörper gegen TNF oder ein TNF bindendes Fragment desselben und ein Xanthin-Derivat als Kombinationspräparat zur gleichzeitigen kombinierten, gleichzeitigen getrennten oder aufeinanderfolgenden therapeutischen Verwendung enthält.

2. Pharmazeutisches Produkt nach Anspruch 1 in Form einer pharmazeutischen Zusammensetzung, die den Antikörper gegen TNF oder ein TNF bindendes Fragment desselben und das Xanthin-Derivat in Mischung enthält.

3. Pharmazeutisches Produkt nach Anspruch 2, in welchem das Verhältnis von Xanthin-Derivat zu anti-TNF Antikörperbestandteil im Bereich zwischen 450:1 und 1:10, bezogen auf Gewicht, liegt.

4. Pharmazeutisches Produkt nach einem der Ansprüche 1 bis 3, in welchem das Xanthin-Derivat eine Verbindung der Formel I 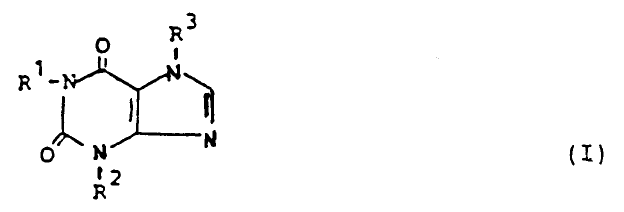 ist, in welcher einer der Reste R¹ und R³ eine geradkettige Alkyl-, ( -1)-Oxoalkyl- oder ( -1)-Hydroxyalkylgruppe mit 3 bis 8 Kohlenstoffatomen bedeutet und die beiden anderen Reste R² und R³ oder R¹ und R² für geradkettige oder verzweigte Alkylgruppen mit 1 bis 8 Kohlenstoffatomen in der Position von R¹ und R³ und mit 1 bis 4 Kohlenstoffatomen in der Position von R² stehen, wobei die Gesamtzahl der Kohlenstoffatome in diesen beiden Alkylsubstituenten 10 nicht übersteigt.

5. Pharmazeutisches Produkt nach einem der Ansprüche 1 bis 3, in welchem das Xanthin-Derivat eine Verbindung der Formel II 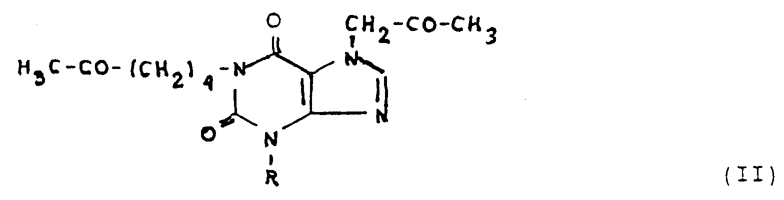 ist, in welcher R für einen Alkylrest mit 1 bis 4 Kohlenstoffatomen steht.

6. Pharmazeutisches Produkt nach einem der Ansprüche 1 bis 3, in welchem das Xanthin-Derivat eine Verbindung der Formel III 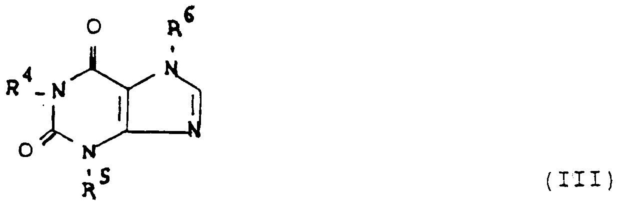 ist, in welcher mindestens einer der Reste R⁴ und R⁶ für eine tertiäre Hydroxylgruppe der Formel IIIa steht 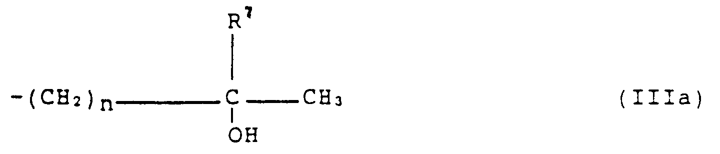 worin R⁷ eine Alkylgruppe mit bis zu 3 Kohlenstoffatomen bedeutet und n für eine ganze Zahl von 2 bis 5 steht und - wenn nur einer der Reste R⁴ oder R⁶ für eine solche tertiäre Hydroxylgruppe der Formel IIIa steht - der andere Rest ein Wasserstoffatom oder einen aliphatischen Kohlenwasserstoffrest R⁸ bedeutet, der bis zu 6 Kohlenstoffatome enthält und dessen Kohlenstoffkette durch bis zu 2 Sauerstoffatome unterbrochen oder durch eine Oxogruppe oder bis zu zwei Hydroxylgruppen substituiert sein kann (in welchem Fall eine in dem Rest R⁸ vorliegende Oxo- oder Hydroxylgruppe vorzugsweise von dem Stickstoff durch mindestens 2 Kohlenstoffatome getrennt ist) und R⁵ für eine Alkylgruppe mit 1 bis 4 Kohlenztoffatomen steht.

7. Pharmazeutisches Produkt nach einem der Ansprüche 4, 5 oder 6, in welchem das Xanthin-Derivat in Form einer Arzneimittelvorstufe oder eines Metabolits der Verbindung der Formel I, II oder III vorliegt.

8. Pharmazeutisches Produkt nach Anspruch 4, in welchem das Xanthin-Derivat
1-Hexyl-3,7-dimethylxanthin,
1-(5-Hydroxyhexyl)-3,7-dimethylxanthin,
3,7-Dimethyl-1-(5-oxohexyl)-xanthin,
7-(5-Hydroxyhexyl)-1,3-dimethylxanthin,
1,3-Dimethyl-7-(5-oxohexyl)-xanthin,
1,3-Di-n-butyl-7-(2-oxopropyl)-xanthin,
1,3-Di-n-butyl-7-(3-oxypropyl)-xanthin,
1-(5-Hydroxyhexyl)-3-methyl-7-propylxanthin,
3-Methyl-1-(oxohexyl)-7-propylxanthin oder
3,7-Dimethyl-1-(5-oxohexyl)-xanthin
umfaßt.

9. Pharmazeutisches Produkt nach Anspruch 6, in welchem das Xanthin-Derivat
7-Ethoxymethyl-1-(5-hydroxy-5-methylhexyl)-3-methylxanthin,
7-Propyl-1-(5-hydroxy-5-methylhexyl)-3-methylxanthin oder
1-(5-Hydroxy-5-methylhexyl)-3-methylxanthin
umfaßt.

10. Pharmazeutisches Produkt nach einem der vorhergehenden Ansprüche, in welchem der anti-TNF Antikörper oder das TNF bindende Fragment desselben ein TNF neutralisierender Antikörper oder ein solches Antikörperfragment ist.

11. Pharmazeutisches Produkt nach einem der vorhergehenden Ansprüche, in welchem der anti-TNF Antikörper oder das Antikörperfragment monospezifisch ist.

12. Pharmazeutisches Produkt nach einem der vorhergehenden Ansprüche, in welchem der anti-TNF Antikörper oder das Anti-körperfragment ein rekombinanter Antikörper oder ein solches Antikörperfragment ist.

13. Pharmazeutisches Produkt nach Anspruch 12, in welchem der anti-TNF Antikörper oder das Antikörperfragment ein humanisierter rekombinanter Antikörper oder ein solches Antikörperfragment ist.

14. Pharmazeutisches Produkt nach einem der vorhergehenden Ansprüche zur Verwendung bei der Besserung der Nebenwirkungen, die bei der TNF-Bildung während einer neoplastischen Therapie auftreten.

15. Pharmazeutisches Produkt nach einem der Ansprüche 1 bis 13 zur Verwendung bei der Eliminierung oder Besserung von mit Schock-Symptomen, die in Zusammenhang mit einer Antilymphozytentherapie stehen.

16. Pharmazeutisches Produkt nach einem der Ansprüche 1 bis 13 zur Verwendung bei der Behandlung von Multiorganverzagen.

17. Pharmazeutisches Produkt nach einem der Ansprüche 1 bis 13 zur Verwendung bei der Behandlung von Sepsis oder septischem/endotoxischem Schock.

18. Verwendung eines Antikörpers gegen TNF oder eines Fragmentes desselben und eines Xanthin-Derivates bei der Herstellung eines pharmazeutischen Produktes zur Verwendung als Kombinationspräparat zur gleichzeitigen kombinierten, gleichzeitigen getrennten oder aufeinanderfolgenden therapeutischen Verwendung.

19. Verfahren zur Herstellung eines pharmazeutsichen Produkts nach Anspruch 2, bei welchem wirksame Mengen eines anti-TNF Antikörpers oder eines Fragmentes desselben mit einem Xanthin-Derivat gemischt werden.

20. Verfahren nach Anspruch 19, bei welchem der Antikörper oder das Fragment desselben und das Xanthin-Derivat auch mit einem pharmazeutisch verwendbaren Exzipient, Verdünnungsmittel oder Träger gemischt werden.

21. Verwendung des pharmazeutischen Produktes nach einem der Ansprüche 1 bis 13 bei der Herstellung einer Zusammensetzung für die Behandlung einer Störung, die mit einem unerwünscht hohen TNF-Spiegel in Zusammenhang steht.

## Revendications

1. Produit pharmaceutique comprenant un anticorps dirigé contre le TNF ou un fragment de celui-ci se fixant au TNF ainsi qu'un dérivé de xanthine sous forme d'une préparation associée pour une utilisation thérapeutique simultanée en association, simultanée séparément ou séquentielle.

2. Produit pharmaceutique selon la revendication 1 sous la forme d'une composition pharmaceutique comprenant un mélange d'anticorps dirigé contre le TNF, ou l'un de ses fragments se fixant au TNF et un dérivé de xanthine.

3. Produit pharmaceutique selon la revendication 2, dans lequel le rapport pondéral du dérivé de xanthine à l'anticorps anti-TNF se situe dans l'intervalle de 450 : 1 à 1 : 10.

4. Produit pharmaceutique selon l'une quelconque des revendications 1 à 3, dans lequel le dérivé de xanthine est un composé de formule I 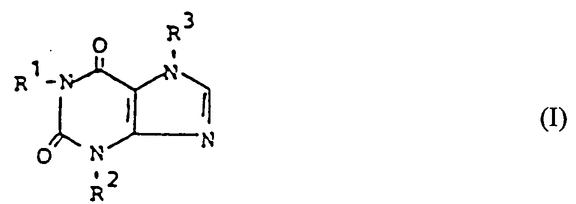 dans laquelle l'un des radicaux R¹ et R³ représente une chaîne alkyle droite, un groupe (ω-1)-oxoalkyle ou (ω-1)-hydroxyalkyle ayant de 3 à 8 atomes de carbone et les deux autres radicaux, R² et R³ ou R¹ et R² représentent des groupes alkyles à chaîne droite ou ramifiée ayant de 1 à 8 atomes de carbone pour R¹ et R³ et de 1 à 4 atomes de carbone pour R², le nombre total d'atomes de carbone dans ces deux substituants alkyles n'étant pas supérieur à 10.

5. Produit pharmaceutique selon l'une quelconque des revendications 1 à 3, dans lequel le dérivé de xanthine est un composé de formule II 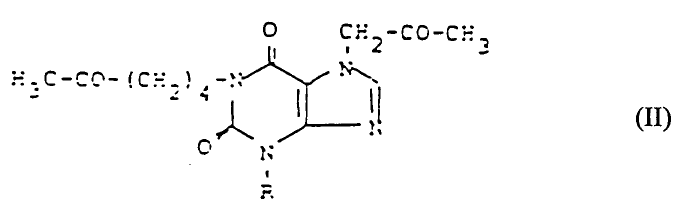 dans laquelle R représente un radical alkyle ayant de 1 à 4 atomes de carbone.

6. Produit pharmaceutique selon l'une quelconque des revendications 1 à 3, dans lequel le dérivé de xanthine est un composé de formule III 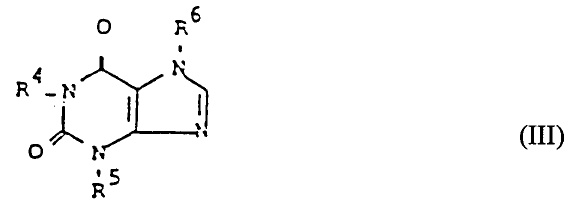 dans laquelle au moins un des radicaux R⁴ et R⁶ représente un groupe hydroxyalkyle tertiaire de formule IIIa 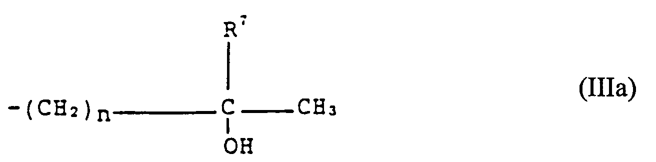 dans laquelle R⁷ désigne un groupe alkyle ayant jusqu'à 3 atomes de carbone et n désigne un nombre entier de 2 à 5 et lorsque l'un seulement des radicaux R⁴ ou R⁶ désigne un tel groupe hydroxyalkyle tertiaire de formule IIIa, l'autre radical représente un atome d'hydrogène ou un radical hydrocarboné aliphatique R⁸ qui possède jusqu'à 6 atomes de carbone et dont la chaîne carbonée peut être interrompue par au plus 2 atomes d'oxygène ou substituée par un groupe oxo ou par au plus 2 groupes hydroxyle (dans ce cas un groupe oxo ou hydroxyle présent dans le radical R⁸ est de préférence séparé de l'azote par au moins 2 atomes de carbone) et R⁵ représente un groupe alkyle ayant de 1 à 4 atomes de carbone.

7. Produit pharmaceutique selon l'une quelconque des revendications 4, 5 ou 6, dans lequel le dérivé de xanthine est sous la forme d'un précurseur de médicament ou d'un métabolite du composé de formule I, II ou III.

8. Produit pharmaceutique selon la revendication 4, dans lequel le dérivé de xanthine comprend :
la 1-hexyl-3,7-diméthylxanthine,
la 1-(5-hydroxyphényl)-3,7-diméthylxanthine,
la 3,7-diméthyl-1-(5-oxohexyl)xanthine,
la 7-(5-hydroxyhexyl)-1,3-diméthylxanthine,
la 1,3-diméthyl-7-(5-oxohexyl)xanthine,
la 1,3-di-n-butyl-7-(2-oxopropyl)xanthine,
la 1,3 di-n-butyl-7-(3-oxybutyl)xanthine,
la 1-(5-hydroxyhexyl)-3-méthyl-7-propylxanthine,
la 3-méthyl-1-(5-oxohexyl)-7-propylxanthine ou
la 3,7-diméthyl-1-(5-oxohexyl)xanthine.

9. Produit pharmaceutique selon la revendication 6, dans lequel le dérivé de xanthine comprend :
la 7-éthoxyméthyl-1-(5-hydroxy-5-méthylhexyl)-3-méthylxanthine,
la 7-propyl-1-(5-hydroxy-5-méthylhexyl)-3-méthylxanthine ou
la 1-(5-hydroxy-5-méthylhexyl)-3-méthylxanthine.

10. Produit pharmaceutique selon l'une quelconque des revendications précédentes, dans lequel l'anticorps anti-TNF ou son fragment se fixant au TNF est un anticorps ou fragment d'anticorps neutralisant le TNF.

11. Produit pharmaceutique selon l'une quelconque des revendications précédentes, dans lequel l'anticorps ou fragment d'anticorps anti-TNF est monospécifique.

12. Produit pharmaceutique selon l'une quelconque des revendications précédentes, dans lequel l' anticorps ou fragment d' anticorps anti-TNF est un anticorps ou fragment d' anticorps recombinant.

13. Produit pharmaceutique selon la revendication 12, dans lequel l'anticorps anti-TNF ou fragment d'anticorps est un anticorps ou fragment d'anticorps recombinant humanisé.

14. Produit pharmaceutique selon l'une quelconque des revendications précédentes, pour une utilisation en vue de la limitation des effets secondaires qui sont associés à la production de TNF au cours de la thérapie néoplasique.

15. Produit pharmaceutique selon l'une quelconque des revendications 1 à 13, pour une utilisation dans l'élimination ou la limitation des symptômes liés au choc qui sont associés à la thérapie antilymphocytaire.

16. Produit pharmaceutique selon l'une quelconque des revendications 1 à 13, pour une utilisation dans le traitement d'une défaillance d'organes multiples.

17. Produit pharmaceutique selon l'une quelconque des revendications 1 à 13, pour une utilisation dans le traitement d'une infection ou d'un choc septique/endotoxique.

18. Utilisation d'un anticorps dirigé contre le TNF ou un fragment de celui-ci ainsi que d'un dérivé de xanthine dans la fabrication d'un produit pharmaceutique pour une utilisation sous forme d'une préparation associée pour une utilisation thérapeutique simultanée en association, simultanée séparément ou séquentielle.

19. Procédé de préparation d'un produit pharmaceutique selon la revendication 2, consistant à mélanger des quantités efficaces d' anticorps anti-TNF ou d'un fragment de celui-ci avec un dérivé de xanthine.

20. Procédé selon la revendication 19, dans lequel l'anticorps ou le fragment de celui-ci et le dérivé de xanthine sont également mélangés avec un excipient, un diluant ou un support pharmaceutiquement acceptables.

21. Utilisation du produit pharmaceutique selon l'une quelconque des revendications 1 à 13, dans la fabrication d'une composition pour le traitement d'un dysfonctionnement associé à un fort niveau indésirable de TNF.
